# EUROPÄISCHE PATENTANMELDUNG

(11) **EP 0 826 655 A1**
(43) Veröffentlichungstag der Anmeldung: **04.03.1998**
(21) Anmeldenummer: 97114736.8
(22) Anmeldetag: 26.08.1997
(51) Int. Cl.: C07C 17/08

(54) **Herstellung von Alkylbromiden aus wässriger Bromwasserstoffsäure und Olefinen**

(30) Priorität: 28.08.1996 DE 19634858
(71) Anmelder: BASF AKTIENGESELLSCHAFT, 67056 Ludwigshafen (DE)
(72) Erfinder: Eiermann, Matthias, Dr., 67117 Limburgerhof (DE); Ebel, Klaus, Dr., 68623 Lampertheim (DE)
(74) Vertreter: Isenbruck, Günter, Dr.

(57) **Zusammenfassung**

In dem Verfahren zur Herstellung von Alkylbromiden aus wäßriger Bromwasserstoffsäure und Olefinen mit Ausnahme konjugierter Diene und Enine, verwendet man als Katalysator eine oder mehrere Elemente M oder Verbindungen der allgemeinen Formel M_{w}LₓA_{y}(H₂O)_{z}, wobei
- M ein oder mehrere Elemente der Gruppen IA, IIA, IIIB, IVB, VB, VIB, VIIB, VIII, IB, IIB, IIIA oder IVA des Periodensystems oder der Lanthaniden,
- L ein ein- oder mehrzähniger Ligand,
- A ein oder mehrere Elemente oder Verbindungen von Elementen der Gruppen VB, VIB, IIIA, IVA, VA, VIA oder VIIA des Periodensystems sind, wobei
- w mindestens 1,
- y mindestens 1,
- x größer oder gleich Null und
- z größer oder gleich 0 sind.

## Beschreibung

Die Erfindung betrifft ein Verfahren zur Herstellung von Alkylbromiden aus wäßriger Bromwasserstoffsäure und Olefinen.

Alkylbromide haben in der organischen Chemie eine große Bedeutung für eine Vielzahl von Anwendungen, beispielsweise für metallorganische Reaktionen.

Allgemein bekannt ist, daß sie aus den entsprechenden Alkoholen durch Umsetzung mit Thionylbromid oder Bromwasserstoffsäure hergestellt werden können. Auch durch Addition von Bromwasserstoff an Olefine sind Alkylbromide zugänglich. Dabei wird Bromwasserstoffgas in einem nichtwäßrigen Medium wie beispielsweise Eisessig eingesetzt. Um die Reaktion des Bromwasserstoffgases zu beschleunigen, sind auch Lewissäuren wie Eisen(III)salze als Katalysatoren eingesetzt worden (vgl. Houben-Weyl, Bd. V/4). Bromwasserstoffgas ist jedoch teuer und in technischem Maßstab nicht beliebig verfügbar.

Die Addition von HBr in konzentrierter wäßriger Lösung an Olefine wie 1-Penten oder 1-Hepten verläuft sehr langsam (M. L. Sherrill, K. E. Mayer, G. F. Walker, J. Am. Chem. Soc. 1934, 56, 926); dabei ist ein extrem großer Überschuß an HBr notwendig, so daß dieser Synthese keine wirtschaftliche Bedeutung zukommt.

Lediglich im Falle der hochreaktiven konjugierten Diene und Enine wurden rasche Umsetzungen beobachtet [W. H. Carothers et al., J. Am. Chem. Soc. 55, S. 787 - 789 (1933)].

Eine weitere Verbesserung der Reaktionsgeschwindigkeit und der Ausbeute kann durch Zusatz von Oniumsalzen (D. Landini, F. Rolla, J. Org. Chem. 1980, 45, 3527), von Radikalinitiatoren (z.B. NE-A 65 03537) oder von stöchiometrischen Mengen Phosphortribromid (SU-A 825 477) erzielt werden; jedoch sind die dafür benötigten Reagenzien teuer und führen im Falle der Radikalinitiatoren bei nicht symmetrisch substituierten Olefinen zu isomeren Produkten. Die Durchführung der Reaktion unter Druck (US 3,679,759) wirkt sich zwar günstig auf die Ausbeute aus, erfordert aber aufwendige Druckapparaturen.

Der Erfindung liegt daher die Aufgabe zugrunde, ein Verfahren zur Herstellung von Alkylbromiden aus Bromwasserstoffsäure und Olefinen bereitzustellen, das in Bezug auf Ausbeute und/oder Geschwindigkeit der Umsetzung verbessert ist. Außerdem soll das Verfahren kostengüstig sein, so daß es auch wirtschaftlich, insbesondere in der Großindustrie, eingesetzt werden kann.

Diese Aufgabe wird dadurch gelöst, daß ein Verfahren zur Herstellung von Alkylbromiden aus wäßriger Bromwasserstoffsäure und Olefinen bereitgestellt wird, das dadurch gekennzeichnet ist, daß als Katalysatoren ein oder mehrere Elemente M oder Verbindungen der allgemeinen Formel M_{W}LₓA_{y}(H₂O)_{z} (I) eingesetzt werden, wobei
- M ein oder mehrere Elemente der Gruppen IB, IIA, IIIB, IVB, VB, VIB, VIIB, VIII, IB, IIB, IIIA oder IVA des Periodensystems oder der Gruppe der Lanthaniden darstellt,
- L ein ein- oder mehrzähniger Ligand oder eine Kombination verschiedener ein- oder mehrzähniger Liganden,
- A ein oder mehrere Element oder Verbindungen von Elementen der Gruppen VB, VIB, IIIA, IVA, VA, VIA oder VIIA des Periodensystems und
- w mindestens 1, x größer oder gleich Null, y mindestens 1 und z größer oder gleich Null sind.

Bevorzugte Ausgestaltungen des erfindungsgemäßen Verfahrens sind Gegenstand der Unteransprüche.

Als Katalysatoren können bei dem erfindungsgemäßen Verfahren beliebige Elemente M oder Verbindungen wie Salze, Koordinationsverbindungen o.ä. der allgemeinen Formel (I) oder deren Mischungen oder Legierungen verwendet werden. Dabei repräsentiert M ein oder mehrere Elemente der Gruppen IA, IIA, IIIB, IVB, VB, VIB, VIIB, VIII, IB, IIB, IIIA oder IVA des Periodensystems oder der Lanthanide. Bevorzugt handelt es sich bei M um ein oder mehrere Elemente der Gruppen IIA, VB, VIB, VIIB, VIII, IB, IIB oder IIIA des Periodensystems. Besonders bevorzugt sind Eisen, Zink oder Aluminium.

Zu den Liganden L. in der Formel (I) gehören insbesondere Carboxylate, Cyanide, Halogenide, Amine, Amide, Phosphine, Phosphonate, Nitrat, Nitrit, Alkoholat, Sulfid, Thiolat, Sulfonat, Olefin, Aryl, Nitril, Imin oder Carbonyl.

A in Formel (I) repräsentiert ein oder mehrere Elemente oder Verbindungen aus Elementen der Gruppen VB, VIB, IIIA, IVA, VA, VIA oder VIIA des Periodensystems. Bevorzugt handelt es sich bei A um Borat, Carboxylat, Carbonat, Cyanid, Amid, Nitrat, Nitrit, Phosphat, Phosphonat, Polyphosphat, Oxid, Hydroxid, Alkoholat, Sulfid, Sulfat, Thiolat, Sulfinat, Sulfonat, Selenid, Halogenid, Halogenat oder Perhalogenat. Insbesondere kann es sich bei dem Polyphosphat um ein Heteropolyphosphat des Molybdäns oder des Wolframs handeln. Besonders bevorzugt handelt es sich bei A um Bromid oder Hydroxid.

Das erfindungsgemäße Verfahren kann bei einem Absolutdruck zwischen 0,001 und 1000, bevorzugt zwischen 1 und 10 bar durchgeführt werden. Insbesondere bevorzugt ist jedoch, wenn das Verfahren bei Normaldruck durchgeführt wird.

Die Reaktionstemperatur liegt zwischen -50 und 400°C, bevorzugt zwischen -20 und 150°C. Besonders bevorzugt aber sind Reaktionstemperaturen zwischen 0°C und dem Siedepunkt der jeweiligen Mischung bei Normaldruck, da somit eine technisch aufwendige Druckfahrweise entfallen kann.

Die Reaktionsführung kann sowohl als kontinuierlicher als auch diskontinuierlicher Prozeß, bevorzugt diskontinuierlich, gestaltet werden.

Bei dem erfindungsgemäßen Verfahren wird das Olefin oder das Olefingemisch mit der wäßrigen Bromwasserstoffsäure ohne oder in Gegenwart eines oder mehrerer weiterer Lösungsmittel, die jedes in Wasser mischbar oder auch nicht mischbar sein können, zur Reaktion gebracht. Bervorzugt wird die Reaktion aber ohne Lösungsmittel oder in Gegenwart eines Alkans oder eines Alkangemischs durchgeführt.

Die Zugabe der Komponenten kann so erfolgen, daß sowohl das Olefin zur Bromwasserstoffsäure als auch umgekehrt die Bromwasserstoffsäure zum Olefin zugefügt wird. Werden ein oder aber mehrere Lösungsmittel verwendet, so können diese wahlweise vorgelegt oder aber einer Vorlage hinzugefügt werden.

Überraschenderweise also wurde gefunden, daß die Additionsreaktion von Bromwasserstoffsäure an Olefine durch Zusatz geeigneter Katalysatoren gemaß Formel (I) auch im wäßrigen Medium in vorteilhafter Weise beschleunigt wird. Dabei kann ein günstigeres Verhältnis der Reaktanden gewählt werden, so daß die auch im technischen Maßstab leicht zugängliche, kostengünstigere wäßrige Bromwasserstoffsäure zur Synthese von Alkylbromiden ausgehend von Olefinen eingesetzt werden kann. Die Bromwasserstoffsäure wird als wäßrige Lösung in einer Konzentration von 0,1 bis 95, bevorzugt von 1 bis 80, ganz besonders bevorzugt von 40 bis 65 Gew.-% eingesetzt.

Wahrend der Umsetzung wird die Mischung vorteilhafterweise durchmischt, insbesondere durch Einsatz einer Rührvorrichtung.

Das bei dem erfindungsgemäßen Verfahren eingesetzte Olefin besitzt die allgemeine Formel R¹(R²)C=C(R³)R⁴, wobei R¹, R², R³ und R⁴ jeweils Wasserstoff, Deuterium oder ein wahlweise mit funktionellen Gruppen substituierter C₁-C₂₀₀-Alkyl-, C₃-C₂₀₀-Cycloalkyl-, C₂-C₂₀₀-Alkenyl-, C₃-C₂₀₀-Cycloalkenyl- oder C₆-C₂₀₀-Aryl-Rest ist. Die funktionelle Gruppe ist unter den erwähnten Resktionsbedingungen chemisch inert. Beispielsweise kann es sich bei ihr um einen Aryl-, einen Halogenid- oder einen Nitro-Rest handeln. R¹, R², R³ und/oder R⁴ sind insbesondere auch Teile gemeinsamer Ringe, und zwar C₁-C₂₀₀-Alkylen, C₃-C₂₀₀-Cycloalkylen, C₂-C₂₀₀-Alkenylen, C₃-C₂₀₀-Cycloalkenylen oder C₆-C₂₀₀-Arylen.

Bevorzugt sind insbesondere C₁-C₅-Alkylenreste, ganz besonders aber C₃-C₄-Alkylenreste. Ganz besonders eignet sich also das erfindungsgemäße Verfahren zur Addition von Bromwasserstoffsäure an Cyclopenten oder Cyclohexen. Ferner können R¹ unter Wegfall von R² sowie R³ unter Wegfall von R⁴ als C₁-C₂₀₀-Alkyliden, C₃-C₂₀₀-Cycloalkyliden, C₂-C₂₀₀-Alkenyliden, C₃-C₂₀₀-Cycloalkenyliden auch doppelt gebunden sein.

Die Reaktion kann mit dem reinen Olefin oder aber auch mit einer Mischung verschiedener Olefine durchgeführt werden, wie sie beispielsweise bei der Dampfspaltung längerkettiger Kohlenwasserstoffe, bei Eliminierungsreaktionen von beispielsweise Alkoholgemischen, bei Pyrolysen von Kohlenwasserstoffen, bei Dehydrierungen von Alkanen oder Hydrierungen von Alkinen, bei der Wittig- oder Wittig-Horner-Reaktion entstehen können.

Ein weiterer Vorteil des erfindungsgemäßen Verfahren liegt in der Möglichkeit zur Ausführung der Reaktion in einem Zweiphasensystem. Eine einfache Abtrennung der nicht umgesetzten wäßrigen Bromwasserstoffsäure vom erhaltenen Alkylbromid nach der Umsetzung wird durch Phasenseparation erreicht. Die wäßrige, in der Regel den Katalysator enthaltende Bromwasserstoffsäure kann nach Abtrennung durch Abdestillieren von Wasser aufkonzentriert und in die Synthese zurückgeführt werden. Aus der nach Abtrennung der wäßrigen Phase verbleibenden organischen Phase kann das Alkylbromid mittels üblicher Verfahrenschritte wie etwa Destillation weiter aufgearbeitet werden.

Bei den erfindungsgemäß eingesetzten Katalysatoren, bei denen es sich um beliebige Elemente M oder Verbindungen der allgemeinen Formel M_{W}L_{X}A_{y}(H₂O)_{z} handelt, die - wie vorstehend bereits erwähnt - definiert ist, ist M bevorzugt ein oder mehrere Elemente der Gruppen IIA, VB, VIB, VIIB, VIII, IB, IIB oder IIIA des Periodersystems, besonders bevorzugt Eisen, Zink oder Aluminium. Von den in Frage kommenden Eisen-, Zink- oder oder Aluminiumverbindungen sind besonders Bromide, Hydroxide geeignet.

Bei dem ein- oder mehrzähnigen Liganden L handelt es sich vorzugsweise um Carboxylat, Cyanid, Halogenid, Amin, Amid, Phosphin, Phosphonat, Nitrat, Nitrit, Alkoholat, Sulfid, Thiolat, Sulfonat, Olefin, Aryl, Nitril, Imin, Carbonyl.

A ist bevorzugt ein Borat, Carboxylat, Carbonat, Cyanid, Amid, Nitrat, Nitrit, Phosphat, Phosphonat, Polyphosphat, Heteropolyphosphat, insbesondere des Molybdäns oder des Wolframs, Oxid, Hydroxid, Alkoholat, Sulfid, Sulfat, Thiolat, Sulfinat, Sulfonat, Selenid, Halogenid, Halogenat oder Perhalogerat. Besonders bevorzugt sind Bromide, Hydroxide.

Der Katalysator kann in reiner Form, in Lösung oder auch auf einem Trägermaterial eingesetzt werden. Als Träger eignen sich insbesondere organische Harze wie beispielsweise Ionentauscherharze, Molekularsiebe, wie beispielsweise Montmorillonite, Kaolinite und Bentonite oder auch Zeolithe. Aber auch poröse Materialien aus Kohlenstoff wie beispielweise Aktivkohle sowie Oxide, Sulfate, Sulfide, Hydroxide, Halogenide, Phosphate oder Pyrophosphate von Elementen der Gruppen IA, IIA, IIIB, IVB, VB, VIB, VIIB, VIII, IB, IIB, IIIA oder IVA des Periodensystems oder der Lanthanide können vorteilhaft eingesetzt werden. Der reine oder derart geträgerte Katalysator kann sowohl in Form eines Pulvers, als Splitt oder auch als Formkörper wie beispielsweise in Strangform verwendet werden. Besonders bevorzugt ist der Einsatz in reiner Form.

Das Molverhältnis Olefin: HBr beträgt in der Regel 0,001:1 bis 1000:1, bevorzugt 0,1:1 bis 10:1 und besonders bevorzugt 0,2:1 bis 1:1. Das Molverhältnis zwischen dem eingesetzten Katalysator und dem Olefin liegt zwischen 0,001 und 1000, bevorzugt zwischen 0,01 und 10. Besonders bevorzugt ist ein Pereich zwischen 0,1 und 1 Mol-%.

### Beispiele

### Beispiel 1

136 g Cyclopenten, 91 g Cyclopentan, 689.4 g 47%ige wäßrige HBr und 4,8 g Eisen(III)sulfat wurden 28 h bei 49 bis 64°C gerührt. Der Umsatz an Cyclopenten liegt nach 24 h bei 80 % (GC, FID-Flächen), nach 28 h bei 82 % (GC, FID-Flächen), die Selektivität für Cyclopentylbromid jeweils bei 99%. Die Aufarbeitung erfolgte in bekannter Weise, beispielsweise durch Phasentrennung, Neutralisierung und Trocknung der organischen Phase und anschließende Destillation. Auf diese Weise erhält man beispielsweise aus dem angegebenen Ansatz ca. 200 g reines Cyclopentylbromid.

### Beispiel 2

Es wurde analog zu Beispiel 1 verfahren; statt 4,8 g Eisen(III)sulfat wurden 2,7 g Zinkbromid verwendet. Der Cyclopenten-Umsatz nach 24 h lag bei 82 % (GC, FID-Flächen), die Selektivität bei 99%.

### Beispiel 3

Es wurde analog zu Beispiel 1 verfahren; statt 4,8 g Eisen(III)sulfat wurden 4,1 g Aluminiumsulfat verwendet. Der Cyclopenten-Umsatz nach 24 h lag bei 82 % (GC, FID-Flächen), die Selektivität bei 99%.

### Vergleichsbeispiel 4

14 g 1-Penten und 69 g 47%ige wäßrige HBr wurden analog Beispiel 1 umgesetzt. Nach 24 h bei Rückflußtemperatur unter Atmosphärendruck betrug der Pentenumsatz 1.9 % (GC, FID-Flächen), die Selektivität der Bildung von 2-Brompentan lag bei 79 %.

### Beispiel 5

Es wurde wie in Vergleichsbeispiel 4 verfahren, zusätzlich wurden zu Beginn der Umsetzung 0,27 g Zinkbromid zugesetzt. Nach 24 h betrug der Pentenumsatz 5,3 % (GC, FID-Flächen), die Selektivität der Bildung von 2-Brompentan lag bei 79 %.

## Patentansprüche

1. Verfahren zur Herstellung von Alkylbromiden aus wäßriger Bromwasserstoffsäure und Olefinen mit Ausnahme konjugierter Diene und Enine, dadurch gekennzeichnet, daß man als Katalysator eine oder mehrere Elemente M oder Verbindungen der allgemeinen Formel M_{w}LₓA_{y}(H₂O)_{z} verwendet, wobei
- M ein oder mehrere Elemente der Gruppen IA, IIA, IIIB, IVB, VB, VIB, VIIB, VIII, IB, IIB, IIIA oder IVA des Periodensystems oder der Lanthaniden,
- L ein ein- oder mehrzähniger Ligand,
- A ein oder mehrere Elemente oder Verbindungen von Elementen der Gruppen VB, VIB, IIIA, IVA, VA, VIA oder VIIA des Periodensystems sind, wobei
- w mindestens 1,
- y mindestens 1,
- x größer oder gleich Null und
- z größer oder gleich 0 sind.

2. Verfahren nach Anspruch 1, dadurch gekennzeichnet, daß M ein oder mehrere Elemente der Gruppen IIA, VB, VIB, VIIB, VIII, IB, IIB oder IIIA des Periodensystems, bevorzugt Eisen, Zink oder Aluminium ist.

3. Verfahren nach einem der Ansprüche 1 oder 2, dadurch gekennzeichnet, daß L Carboxylat, Cyanid, Halogenid, Amin, Amid, Phosphin, Posphonat, Nitrat, Nitrit, Alkoholat, Sulfit, Thiolat, Sulfonat oder ein Rest ist, der aus der Gruppe von Olefin, Aryl, Nitril, Imin und Carbonyl ausgewählt ist.

4. Verfahren nach einem der vorherigen Ansprüche, dadurch gekennzeichnet, daß A aus der Gruppe ausgewählt ist, umfassend Carboxylat, Carbonat, Cyanid, Amid, Nitrat, Nitrit, Phosphat, Phosphonat, Polyphosphat, Heteropolyphosphat des Molybdäns oder des Wolframs, Oxid, Hydroxid, Alkoholat, Sulfid, Sulfat, Thiolat, Sulfinat, Sulfonat, Selenid, Halogenid, Halogenat und Perhalogenat, bevorzugt Bromid und Hydroxid.

5. Verfahren nach einem der vorherigen Ansprüche, dadurch gekennzeichnet, daß die Bromwasserstoffsäure in einer Konzentration von 1 bis 80 %, bevorzugt von 40 bis 65% eingesetzt wird.

6. Verfahren nach einem der vorherigen Ansprüche, dadurch gekennzeichnet, daß als Olefin eine Verbindung der allgemeinen Formel R¹(R²)C=C(R³)R⁴ eingesetzt wird, wobei R¹, R², R³ und R⁴ jeweils unabhängig voneinander Wasserstoff, Deuterium oder ein unsubstituiertes oder substituiertes C₁-C₂₀₀-Alkyl, C₃-C₂₀₀-Cycloalkyl, C₂-C₂₀₀-Alkenyl, C₃-C₂₀₀-Cycloalkenyl oder C₆-C₂₀₀-Aryl sind.

7. Verfahren nach Anspruch 6, dadurch gekennzeichnet, daß das C₁-C₂₀₀-Alkyl, C₃-C₂₀₀-Cycloalkyl, C₂-C₂₀₀-Alkenyl, C₃-C₂₀₀-Cycloalkenyl oder C₆C₂₀₀-Aryl mit Aryl-, Halogenid- oder Nitroresten substituiert ist.

8. Verfahren nach Anspruch 7, dadurch gekennzeichnet, daß R¹, R², R³ und/oder R⁴ C₃- bis C₄-Alkylenreste sind.

9. Verfahren nach einem der vorherigen Ansprüche, dadurch gekennzeichnet, daß der Katalysator auf einem Trägermaterial aufgebracht ist.

10. Verfahren nach einem der vorherigen Ansprüche, dadurch gekennzeichnet, daß das Trägermaterial ein organisches Harz, ein Molekularsieb oder ein poröses Material ist.
